# EUROPEAN PATENT SPECIFICATION

(11) **EP 2 334 635 B1**
(45) Date of publication and mention of the grant of the patent: **22.03.2017**
(21) Application number: 09791559.9
(22) Date of filing: 17.08.2009
(51) Int. Cl.: C07C 231/02, C07C 233/09

(54) **PROCESS FOR PRODUCING N-ALKYL (ALKYL)ACRYLAMIDES**
VERFAHREN ZUR HERSTELLUNG VON N-ALKYL (ALKYL)ACRYLAMIDEN
PROCÉDÉ DE PRODUCTION DE N-ALKYL (ALKYL) ACRYLAMIDES

(30) Priority: 19.08.2008 US 194267
(43) Date of publication of application: 22.06.2011
(73) Proprietor: Nalco Company, Naperville, IL 60563-1198 (US)
(72) Inventor: MORRIS, John, D., Naperville IL 60540 (US)
(74) Representative: Wilson, Gary
(86) International application number: PCT/US2009/053973
(87) International publication number: WO 2010/021956

(56) References cited:
- US-A- 2 311 548
- US-A- 2 773 063
- US-A- 4 228 102
- DROBNIK J ET AL: "ENZYMATIC CLEAVAGE OF SIDE CHAINS OF SYNTHETIC WATER-SOLUBLE POLYMERS" MAKROMOLEKULARE CHEMIE, MACROMOLECULAR CHEMISTRY AND PHYSICS, HUTHIG UND WEPF VERLAG, BASEL, CH, vol. 177, 1 January 1976 (1976-01-01), pages 2833-2848, XP000943422 ISSN: 0025-116X
- YOSHIO IWAKURA, FUJIO TODA, HIDEAKI SUZUKI: "Synthesis of N-[1-(1-substituted 2-oxopropyl)]acrylamides and -methylacrylamides. Isolation and some reactions of intermediates of the Dakin-West reaction" THE JOURNAL OF ORGANIC CHEMISTRY, vol. 32, no. 2, February 1967 (1967-02), pages 440-443, XP002567337
- YOSHIO IWAKURA, FUJIO TODA, HIDEAKI SUZUKI: "Synthesis and polymerization of N-[1-(1-substituted-2-oxopropyl)]acrylamid es and -methacrylamides. copolymerization of these monomers with styrene and substituent effects" JOURNAL OF POLYMER SCIENCE PART A-1: POLYMER CHEMISTRY, vol. 5, no. 7, July 1967 (1967-07), pages 1599-1607, XP002567338
- Carey, Francis A., Sundberg, Richard J.: "Advanced Organic Chemistry" 2007, Springer Science+Business Media, LLC , XP002567339 ISBN: 9780387683546pages 252-252, page 252, paragraph 4
- Jonathan D. Toker ET AL: "Exploring the Scope of the 29G12 Antibody Catalyzed 1,3-Dipolar Cycloaddition Reaction", The Journal of Organic Chemistry, vol. 70, no. 20, 1 September 2005 (2005-09-01), pages 7810-7815, XP055195563, ISSN: 0022-3263, DOI: 10.1021/jo050410b
- Ana Erceg Kuzmic ET AL: "Nanoporous Crosslinked Copolymers Prepared by Thermal Degradation of Poly(Methacryl- N , N '-diisopropylurea-co-ethylene Glycol Dimethacrylate)", JOURNAL OF MACROMOLECULAR SCIENCE , Part A - PURE AND APPLIED CHEMISTRY., vol. 41, no. 10, 31 December 2004 (2004-12-31), pages 1087-1094, XP55263602, US ISSN: 1060-1325, DOI: 10.1081/MA-200026543

## Description

### BACKGROUND

The present disclosure relates generally to N-alkyl (alkyl)acrylamides. More specifically, the present disclosure relates to methods of producing N-alkyl (alkyl)acrylamides and using the N-alkyl (alkyl)acrylamides.

Anhydrides are relatively reactive in the presence of nucleophiles such as amines, hydroxides, alkoxides, etc. The reactions of acrylic anhydrides and (meth)acrylic anhydrides with a nucleophile such as an amine produces the corresponding acrylic or (meth)acrylic acid, and the subsequent nucleophilic addition product as the other monomer. The resulting N-alkyl (alkyl)acrylamides can be useful as building blocks for polymeric gas hydrate inhibitors. Nevertheless, these reactions can have associated problems related to purification of the final products and the control over side reactions.

US2311548A discloses processes to prepare resinous polymers and interpolymers of N-alkylamides of alpha-methylene aliphatic monocarboxylic acids. Particularly, there is disclosed a product of the interpolymerization of 20 parts of N-methylmethacrylamide with 80 parts of methyl methacrylate.
Drobik et al., as published in Makromol. Chem. 177, pp. 2833-2848 (1976) discloses the preparation of water-soluble polymers based on poly[N-(2-hydroxypropyl)methacrylamide], and includes description of preparative processes for various intermediates including N-methacryloyl-amino acid.
Iwakura et al., as published in The Journal of Organic Chemistry, vol. 32, no.2, pp. 440-443 (1967), discloses the use of the Dakin-West reaction in the synthesis of N-acyl-α-amino ketones. Iwakura et al., as published in Journal of Polymer Science: Part A-1, vol. 5, no.7, pp. 1599- 1607 (1967) discloses the synthesis and polymerization of N-[-(1-substituted-2-oxopropyl)] acrylamides and methacrylamides and the copolymerization of these monomers with styrene.
Toker et al., as published in The Journal of Organic Chemistry, vol. 70, no. 20, pp.7810-7815, US44228102A and US2773063A all disclose methods for the preparation of (meth)acrylamide derivatives of isopropylamine.
The reaction of isopropylamine with methacrylic anhydride in ether in the presence of hydroquinone is disclosed in the Journal of Macromolecular Science, vol. 41, mo. 10, 31 December 2014, pages 1087 to 1094.

### SUMMARY

The present disclosure relates to methods of producing N-alkyl (alkyl)acrylamides. In a general embodiment, the present invention provides a method of producing an N-alkyl methacrylamide as defined in claim 1. The method comprises providing an aqueous solution comprising isopropylamine and adding to the aqueous solution a base and an amount of methacrylic anhydride to form a precipitated N-alkyl methacrylamide.

The method further comprises filtering the aqueous solution to remove the precipitated N-alkyl methacrylamide from the aqueous solution. The precipitated N-alkyl methacrylamide that was filtered can further be washed to remove any contaminant from the N-alkyl methacrylamide.

In an embodiment, the removed contaminant is substantially methacrylic acid salt coproduct.

In an embodiment of the invention, the methacrylic anhydride and the base are added to the aqueous solution at a temperature of below 30 °C or at a temperature of 20 to 30°C.

In an embodiment of the invention, the base is sodium hydroxide, potassium hydroxide, ammonium hydroxide or a combination thereof.

In the present invention, the precipitated N-alkyl methacrylamide has the following structure: 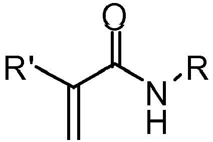 wherein R' is methyl and R is isopropyl.

An advantage of the present disclosure is to provide an improved method of making N-alkyl methacrylamides.

Additional features and advantages are described herein, and will be apparent from the following Detailed Description.

### DETAILED DESCRIPTION

The present disclosure relates to the field of producing N-alkyl (alkyl)acrylamides. As described herein, an aqueous process is used to produce an N-alkyl methacrylamide from the reaction of an N-alkyl amine (isopropylamine) with an anhydride (methacrylic anhydride). The N-alkyl methacrylamide thus produced is of suitable quality for use in subsequent free radical polymerization reactions and other similar chemistries.

Some advantages of the method of the present disclosure over previous procedures described in the literature relate to the ease of purification of the method and the control over side reactions. In addition, the non-volatility of the solvent (e.g. water) provides another advantage during the reactions. The reaction can be performed at relatively low temperatures and thus the desired addition product is favored over other potential side-reactions such as Michael-type reactions that might occur between amines and acrylics at elevated temperatures.

As used herein, "alkyl" means iso-propyl.

The present disclosure provides a method of producing an N-alkyl methacrylamide. The method comprises providing an aqueous solution comprising isopropylamine and adding to the aqueous solution a base and methacrylic anhydride. The base and the anhydride can be added to the aqueous solution while stirring the aqueous solution. The aqueous solution of a base and the anhydride can be added sequentially or simultaneously. Alternatively, an aqueous solution of base and methacrylic anhydride can be prepared and then the N-alkyl amine added. The produced N-alkyl methacrylamide monomer, formed by reaction between the amine and the anhydride, can precipitate from the reaction mixture as a relatively pure product.

An approximate equimolar amount of both the methacrylic anhydride and a base can be added to the aqueous solution. The base can any suitable base such as, for example, sodium hydroxide, potassium hydroxide, or ammonium hydroxide. The methacrylic anhydride and the base can be added to and/or mixed in the aqueous solution at a temperature of below 30 °C. The methacrylic anhydride and the base are added to and/or mixed in the aqueous solution at a temperature ranging from 20 °C to 30 °C.

The method further comprises filtering the aqueous solution to remove the precipitated N-alkyl methacrylamide from the aqueous solution. The method can further comprise washing the precipitated N-alkyl methacrylamide that was filtered to remove any contaminant from the N-alkyl methacrylamide. For example, the solid/precipitated product can be filtered and washed with water to remove any methacrylic acid salt coproduct contaminating the product.

The methacrylic anhydride is an anhydride of formula 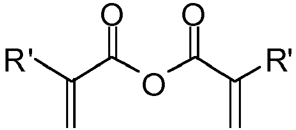 where R' is Me.

The methacrylic anhydride raw material is commercially available or can be made using any of a number of known processes.

The N-alkyl amine is 2-propylamine.

In the present invention, the N-alkyl methacrylamide has the following structure: 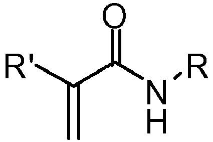 wherein R' is methyl and R is isopropyl.

The present disclosure provides a method of producing an N-alkyl methacrylamide The method comprises providing an aqueous solution comprising an N-alkyl amme and adding to the aqueous solution a base and an amount ofmethacrylic anhydride to form a precipitated N-alkyl methacrylamide. The method comprises filtering the aqueous solution to remove the precipitated N-alkyl methacrylamide from the aqueous solution and can further comprise washing the precipitated N-alkyl methacrylamide that was filtered to remove any contaminant

### EXAMPLES

By way of example and not limitation, the following example is illustrative of the present invention.

### Example 1

The following experiment utilized aqueous reaction conditions to produce N-isopropyl methacrylamide (IPMA):

32 grams of water was added to a 250 mL, three-necked resin flask equipped with a mechanical stirrer, condenser and thermocouple. The water was cooled to 5-9 °C via an ice bath. 6 grams of isopropylamine was slowly added to the cooled water while mixing.

The following components were added to the cooled, stirring isopropylamine solution separately and simultaneously via two separate syringe pumps: 1) methacrylic anhydride (16.53 g of 94% purity) and 2) a 50 wt.% aqueous solution of sodium hydroxide (8.12 g). The methacrylic anhydride and sodium hydroxide solution were each added over a one hour period while keeping the reaction temperature below 30 °C. During this time, a precipitate formed in the reaction mixture. After all of the reagents were added, the reaction mixture was allowed to stir for one additional hour, and then the reactor contents were filtered. After drying the recovered solid under vacuum, 10.7 g product (IPMA) was obtained. Nuclear magnetic resonance (NMR) analysis revealed a product comprised of 93 % IPMA, 4% water, and 3% impurities.

## Claims

1. A method of producing an N-alkyl methacrylamide, the method comprising:
providing an aqueous solution comprising isopropylamine;
adding to the aqueous solution a base and an amount of methacrylic anhydride to form a precipitated N-alkyl methacrylamide; and
filtering the aqueous solution to remove the precipitated N-alkyl methacrylamide from the aqueous solution.

2. The method of Claim 1, wherein the precipitated N-alkyl methacrylamide has the following structure: 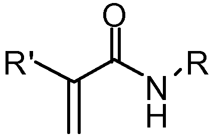 wherein R' is methyl and R is isopropyl.

3. The method of Claim 1, wherein an equimolar amount of the methacrylic anhydride and the base is added to aqueous solution.

4. The method of Claim 1, wherein the methacrylic anhydride and the base are added to the aqueous solution at a temperature of below 30 °C.

5. The method of Claim 1, wherein the methacrylic anhydride and the base are added to the aqueous solution at a temperature of 20 to 30 °C.

6. The method of Claim 1, wherein the base is selected from the group consisting of sodium hydroxide, potassium hydroxide, ammonium hydroxide and combinations thereof.

7. The method of Claim 1, further comprising washing the precipitated N-alkyl methacrylamide that was filtered to remove any contaminant from the N-alkyl methacrylamide.

8. The method of Claim 1, wherein the base is aqueous sodium hydroxide.

9. The method of Claim 8, wherein the methacrylic anhydride and the aqueous sodium hydroxide are added simultaneously to an aqueous solution of isopropylamine.

## Patentansprüche

1. Verfahren zum Herstellen eines N-Alkyl-Methacrylamids, das Verfahren umfassend:
Bereitstellen einer wässrigen Lösung umfassend Isopropylamin;
Hinzufügen einer Base und einer Menge von Methacrylanhydrid zu der wässrigen Lösung, um ein ausgefälltes N-Alkyl-Methacrylamid zu bilden; und
Filtern der wässrigen Lösung, um das ausgefällte N-Alkyl-Methacrylamid aus der wässrigen Lösung zu entfernen.

2. Verfahren nach Anspruch 1, wobei das ausgefällte N-Alkyl-Methacrylamid die folgende Struktur hat: 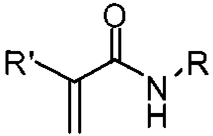 wobei R' Methyl ist und R Isopropyl ist.

3. Verfahren nach Anspruch 1, wobei eine äquimolare Menge des Methacrylanhydrids und der Base zu wässriger Lösung hinzugefügt wird.

4. Verfahren nach Anspruch 1, wobei das Methacrylanhydrid und die Base bei einer Temperatur unter 30 °C zu der wässrigen Lösung hinzugefügt wird.

5. Verfahren nach Anspruch 1, wobei das Methacrylanhydrid und die Base bei einer Temperatur von 20 bis 30 °C zu der wässrigen Lösung hinzugefügt wird.

6. Verfahren nach Anspruch 1, wobei die Base ausgewählt ist von der Gruppe bestehend aus Natriumhydroxid, Kaliumhydroxid, Ammoniumhydroxid und Kombinationen davon.

7. Verfahren nach Anspruch 1, ferner umfassend ein Waschen des ausgefällten N-Alkyl-Methacrylamids, das gefiltert wurde, um jede Verunreinigung von dem N-Alkyl-Methacrylamid zu entfernen.

8. Verfahren nach Anspruch 1, wobei die Base wässriges Natriumhydroxid ist.

9. Verfahren nach Anspruch 8, wobei das Methacrylanhydrid und das wässrige Natriumhydroxid gleichzeitig zu einer wässrigen Lösung von Isopropylamin hinzugefügt werden.

## Revendications

1. Procédé de production d'un N-alkyl méthacrylamide, le procédé comprenant :
la fourniture d'une solution aqueuse comprenant de l'isopropylamine ;
l'ajout à la solution aqueuse d'une base et d'une quantité d'anhydride méthacrylique pour former un N-alkyl méthacrylamide précipité ; et
la filtration de la solution aqueuse pour enlever le N-alkyl méthacrylamide précipité de la solution aqueuse.

2. Procédé selon la revendication 1, ledit N-alkyl méthacrylamide précipité ayant la formule suivante : 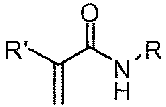 dans laquelle R' représente un groupe méthyle et R représente un groupe isopropyle.

3. Procédé selon la revendication 1, une quantité équimolaire de l'anhydride méthacrylique et de la base étant ajoutée à la solution aqueuse.

4. Procédé selon la revendication 1, ledit anhydride méthacrylique et ladite base étant ajoutés à la solution aqueuse à une température inférieure à 30°C.

5. Procédé selon la revendication 1, ledit anhydride méthacrylique et ladite base étant ajoutés à la solution aqueuse à une température de 20 à 30°C.

6. Procédé selon la revendication 1, ladite base étant choisie dans le groupe constitué par l'hydroxyde de sodium, l'hydroxyde de potassium, l'hydroxyde d'ammonium et des combinaisons de ceux-ci.

7. Procédé selon la revendication 1 comprenant en outre le lavage du N-alkyl méthacrylamide précipité qui a été filtré pour enlever tout contaminant du N-alkyl méthacrylamide.

8. Procédé selon la revendication 1, ladite base étant l'hydroxyde de sodium aqueux.

9. Procédé selon la revendication 8, ledit anhydride méthacrylique et ledit hydroxyde de sodium aqueux étant ajoutés en même temps à une solution aqueuse d'isopropylamine.
